(19) Europäisches Patentamt
European Patent Office
Office européen des brevets

(11) **EP 2 856 997 A2**

## (12) EUROPEAN PATENT APPLICATION

(43) Date of publication:
**08.04.2015 Bulletin 2015/15**

(51) Int Cl.:
**A61H 1/02** (2006.01)     **A61H 3/00** (2006.01)

(21) Application number: **14184463.9**

(22) Date of filing: **11.09.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **17.09.2013   JP 2013191381**

(71) Applicant: **Kabushiki Kaisha Yaskawa Denki Kitakyushu-shi, Fukuoka 806-0004 (JP)**

(72) Inventors:
- **Nakanishi, Yoshie**
  **Kitakyushu-shi, Fukuoka 806-0004 (JP)**
- **Nagata, Hideo**
  **Kitakyushu-shi, Fukuoka 806-0004 (JP)**
- **Osaki, Mikio**
  **Kitakyushu-shi, Fukuoka 806-0004 (JP)**

(74) Representative: **Viering, Jentschura & Partner Patent- und Rechtsanwälte**
**Grillparzerstrasse 14**
**81675 München (DE)**

(54) **Movement assist device**

(57)    A movement assist device (1) includes: a motor (4) including a stator (2) and a rotator (3); two arm members (5, 6) connected to each of the stator and the rotator and configured to be connected to a predetermined part of a body of an equipped person (M); a signal accepting unit (53) configured to accept a trigger signal that triggers a driving of the motor; and a driving control unit (61) configured to drive the motor so that the two arm members expand in response to a trigger that the signal accepting unit accepts a first one of the trigger signal.

*FIG. 1A*

**(Cont. next page)**

EP 2 856 997 A2

# FIG. 1B

## Description

CROSS-REFERENCE TO RELATED APPLICATION

BACKGROUND

1. Technical Field

Disclosed embodiments relate to a movement assist device.

2. Related Art

Japanese Patent Application Laid-open Publication No. S61-228854 discloses dynamic force equipment such as an artificial leg. In this dynamic force equipment, the length of the connection member that connects two hydraulic actuators provided as joints is adjustable. Furthermore, inside the connection member, an oil passage for actuating the hydraulic actuator is provided.

When the dynamic force equipment of the above-described conventional art is used to assist a disabled person having a handicap in the physical function, it is necessary to change the operating timing and the like of the actuator according to the physical function (or the physical state) of the user. In the above-described conventional art, however, this point is not considered and therefore it cannot provide the assist according to the physical function of the equipped person.

One aspect of the embodiments has been made in view of the above problem, and one of its purposes is to provide a movement assist device that allows for the assist according to the physical function of the equipped person.

SUMMARY

A movement assist device includes: a motor including a stator and a rotator; two arm members connected to each of the stator and the rotator and configured to be connected to a predetermined part of a body of an equipped person; a signal accepting unit configured to accept a trigger signal that triggers a driving of the motor; and a driving control unit configured to drive the motor so that the two arm members expand in response to a trigger that the signal accepting unit accepts a first one of the trigger signal.

BRIEF DESCRIPTION OF DRAWINGS

FIG. 1A and FIG. 1B are perspective views illustrating examples of movement assist devices of one embodiment that is attached to a person wearing hip joint equipment;
FIG. 2 is a perspective view illustrating an example of a motor and two arm members of the movement assist device;
FIG. 3 is a perspective view illustrating an example of an attachment structure of the motor and two arm members to the hip joint equipment;
FIG. 4A and FIG. 4B are sectional views illustrating an example of sectional structures of connection parts of the two arm members and the hip joint equipment, respectively;
FIG. 5 is a schematic view illustrating an example of a manual switch of the movement assist device;
FIG. 6 is a schematic view illustrating an example of a sensor unit of the movement assist device;
FIG. 7 is a functional block diagram illustrating an example of a functional configuration of the movement assist device;
FIG. 8 is a schematic view illustrating an example of the sensor unit of the movement assist device when pressure-sensitive sensors are mounted on both legs;
FIG. 9 is a functional block diagram illustrating an example of the functional configuration of the movement assist device when the pressure-sensitive sensors are mounted on both legs;
FIG. 10 is a perspective view illustrating an example of the movement assist device when applied to knee joint equipment;
FIG. 11 is a perspective view illustrating an example of the movement assist device when applied to ankle joint equipment;
FIG. 12 is a perspective view illustrating an example of the movement assist device when applied to shoulder joint equipment;
FIG. 13 is a perspective view illustrating an example of the movement assist device when applied to elbow joint equipment; and
FIG. 14 is a perspective view illustrating an example of the movement assist device when applied to wrist joint equipment.

DETAILED DESCRIPTION

In the following detailed description, for purpose of explanation, numerous specific details are set forth in order to provide a thorough understanding of the disclosed embodiments. It will be apparent, however, that one or more embodiments may be practiced without these specific details. In other instances, well-known structures and devices are schematically shown in order to simplify the drawing.

A movement assist device according to a view point of an embodiment includes: a motor including a stator and a rotator; two arm members connected to each of the stator and the rotator and configured to be connected to a predetermined part of a body of an equipped person; a signal accepting unit configured to accept a trigger signal that triggers a driving of the motor; and a driving control unit configured to drive the motor so that the two arm members expand in response to a trigger that the signal accepting unit accepts a first one of the trigger signal.

This movement assist device may be a movement assist device that assists movement of an equipped person, for example.

A movement assist device according to another view point of the embodiment includes; a motor including a stator and a rotator; two arm members connected to each of the stator and the rotator and configured to be connected to a predetermined part of a body of an equipped person; means for accepting a trigger signal that triggers a driving of the motor; and means for driving the motor so that the two arm members expand in response to a trigger that the means for accepting the trigger signal accepts a first one of the trigger signal.

According to the movement assist device of the embodiment, the assist according to the physical function (or the physical state) of the equipped person can be achieved.

One embodiment will be described below by referring to the drawings. The present embodiment is an example in which the movement assist device is applied to assist the movement of the hip joint.

< General configuration of the movement assist device >

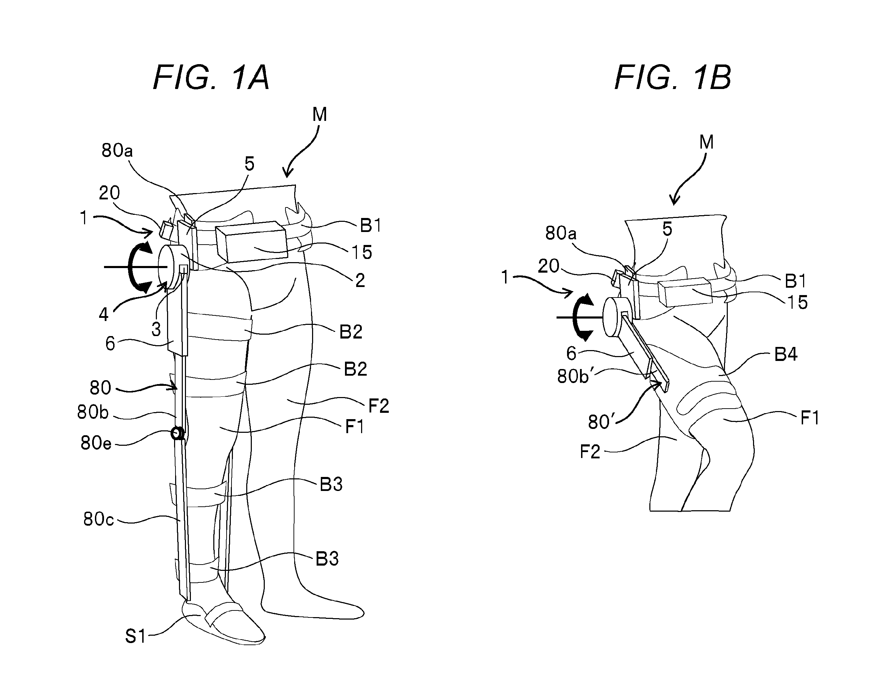

In the examples illustrated in FIG. 1A and FIG. 1B, a movement assist device 1 of the present embodiment assists the movement of a hip joint of an equipped person M who wears hip joint equipment 80 or 80' on a right leg F1. The movement assist device 1 is applicable to both of the two hip joint equipment 80 and 80' illustrated in FIG. 1A and FIG. 1B. It is noted that illustration of a first connector 7 and a second connector 8 is omitted in FIG. 1A and FIG. 1B.

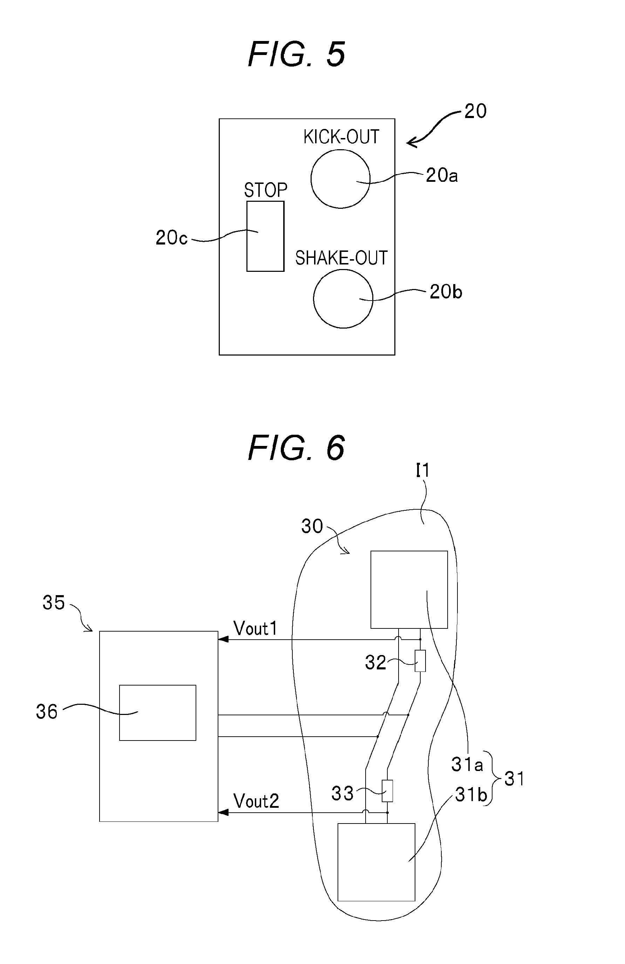

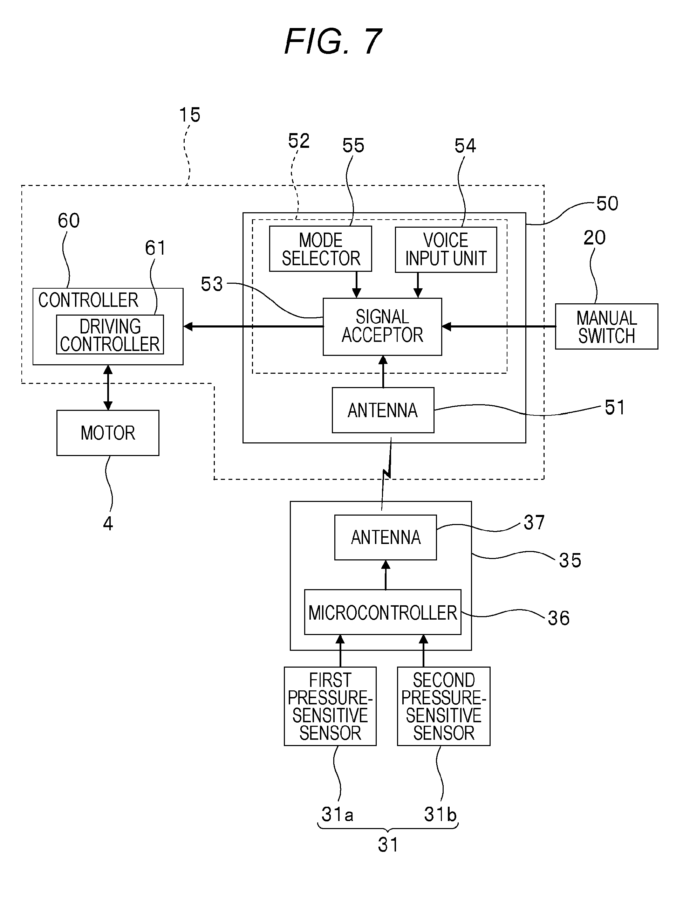

The movement assist device 1 has a motor 4, a first arm member 5, a second arm member 6, a sensor unit 30 (see FIG. 6 described later), a manual switch 20, a control module 50 (see FIG. 7 described later), a controller 60 (see FIG. 7 described later), and a not-shown microphone. The first arm member 5 is connected to a stator 2 of the motor 4. The second arm member 6 is connected to a rotator 3 of the motor 4.

< Hip joint equipment >

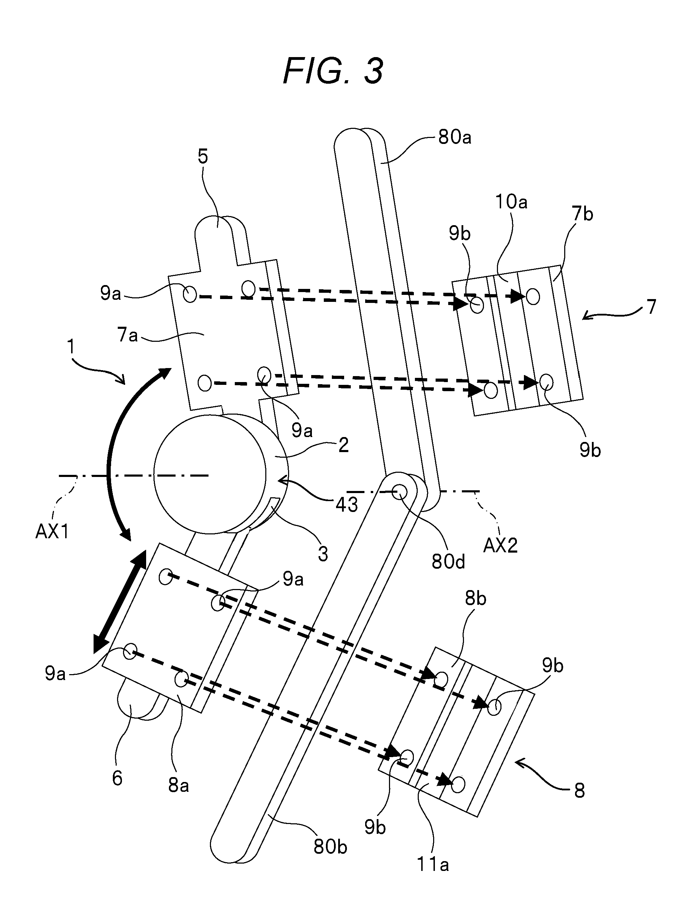

The hip joint equipment 80 illustrated in FIG. 1A is utilized to fix a knee joint, for example. The hip joint equipment 80 includes a waist frame 80a, a femur frame 80b, and a cruris frame 80c. The waist frame 80a is attached to the right side of the waist via a waist band B1 mounted around the waist of the equipped person M. The upper end of the femur frame 80b is connected to the lower part of the waist frame 80a in a rotatable manner by a connection part 80d (see FIG. 3 described later). The femur frame 80b is attached along the longitudinal direction of the femur of the right leg F1 via leg bands B2 mounted on two parts, namely, the upper part and the lower part in the femur of the right leg F1. The femur frame 80b is revolved with respect to the waist frame 80a in response to the walking movement of the equipped person M and therefore is able to expand and bend with respect to the waist frame 80a. It is noted that the term "expand" refers to the revolution of the femur frame 80b (or the relative revolution of the waist frame 80a and the femur frame 80b) to the direction so that the angle between the waist frame 80a and the femur frame 80b (the angle of the foreside of the body) increases. In contrast, the term "bend" refers to the revolution of the femur frame 80b (or the relative revolution of the waist frame 80a and the femur frame 80b) to the direction so that the above-described angle decreases.

The upper end of the cruris frame 80c is connected to the lower end of the femur frame 80b by a connection part 80e. The cruris frame 80c is able to be fixed or revolved with respect to the femur frame 80b. The cruris frame 80c is attached along the longitudinal direction of the cruris of the right leg F1 via leg bands B3 mounted on two parts, namely, the upper part and the lower part in the cruris of the right leg F1 of the equipped person M.

It is noted that, while the depiction is omitted, the backside of the hip joint equipment 80 is provided with a curve frame made of metal, plastic, or the like partially covering the rear half part of the femur and the cruris. This curve frame supports the lower limb. The front face of this curve frame is fixed by the above-described bands B2, B3, and the like.

The hip joint equipment 80' illustrated in FIG. 1B is utilized when it is not necessary to fix the knee joint, for example. The hip joint equipment 80' has the same configuration as the above-described hip joint equipment 80 except that it does not have the cruris frame 80c of the hip joint equipment 80 as illustrated in FIG. 1A and that a short femur frame 80b' is connected to the waist frame 80a. The femur frame 80b' is attached along the longitudinal direction of the femur of the right leg F1 via a supporter band B4 mounted on the femur of the right leg F1 of the equipped person M.

The motor 4, the first arm member 5, and the second arm member 6 of the movement assist device 1 form a movement mechanical part attached to the hip joint equipment 80 (80'). The first arm member 5 is connected to the waist of the equipped person M via the waist frame 80a of the hip joint equipment 80 (80'). The second arm member 6 is connected to the femur of the right leg F1 of the equipped person M via the femur frame 80b (80b'). Further, the sensor unit 30 (see FIG. 6) is arranged to a footwear S1 in the right leg F1 side. The manual switch 20 is arranged to the waist band B1 (the right side of the waist of the equipped person M in the examples illustrated in FIG. 1A and FIG. 1B). Further, the control module 50 and the controller 60 (see FIG. 7) are accommodated in a control box 15 attached to the waist band B1 (in the foreside of the waist of the equipped person M in the examples illustrated in FIG. 1A and FIG.

1B). The microphone (not shown) is set to the waist of the equipped person M via the waist band B1, for example. It is noted that the control module 50 and/or the controller 60 may be fixed to other position than the waist of the equipped person M (for example, the chest and the like).

< Connection of the arm members to the hip joint equipment >

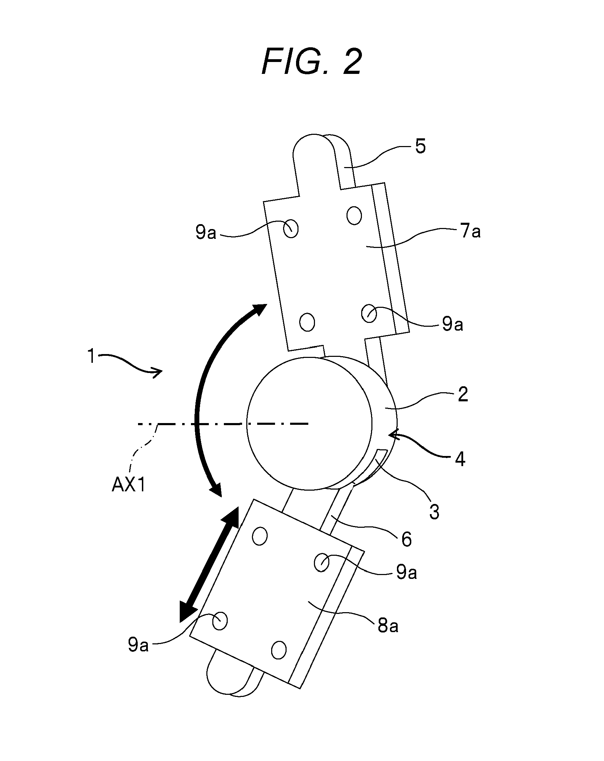

As illustrated in FIG. 2, the first arm member 5 has a plate-like connection plate 7a integrally provided to the first arm member 5 in the intermediate part in the longitudinal direction of the first arm member 5. The connection plate 7a forms one part of the first connector 7 (see FIG. 3 described later). In the connection plate 7a, screw holes 9a piercing through in the thickness direction are provided in multiple positions (four positions near four corners in this example). One end of the first arm member 5 (the lower side in FIG. 2) is fixed to the stator 2 and the first arm member 5 protrudes above the motor 4. The first arm member 5 fixed to the stator 2 revolves within a plane substantially vertical to the rotation axis AX1 of the motor 4.

The second arm member 6 has a slider 8a for attachment. The slider 8a forms one part of the second connector 8 (see FIG. 3 described later). One end of the second arm member 6 (the upper side in FIG. 2) is fixed to the rotator 3 and the second arm member 6 protrudes in the opposite side of the first arm member 5. The slider 8a is formed in a plate-like shape and mounted to the second arm member 6 so as to be able to move in the longitudinal direction of the second arm member 6. In the slider 8a, the screw holes 9a piercing through in the thickness direction are provided in multiple positions (four positions near four corners in this example). The second arm member 6 fixed to the rotator 3 revolves within a plane substantially vertical to the rotation axis AX1 of the motor 4.

As illustrated in FIG. 3, a connection plate 7b forming the other part of the first connector 7 is formed in a plate-like shape similar to the above-described one connection plate 7a. A recess groove 10a for accommodating the waist frame 80a is provided on the surface of the other connection plate 7b facing the one connection plate 7a. Further, screw holes 9b corresponding to the screw holes 9a of the one connection plate 7a are provided in multiple positions of the connection plate 7b (four positions near four corners in this example).

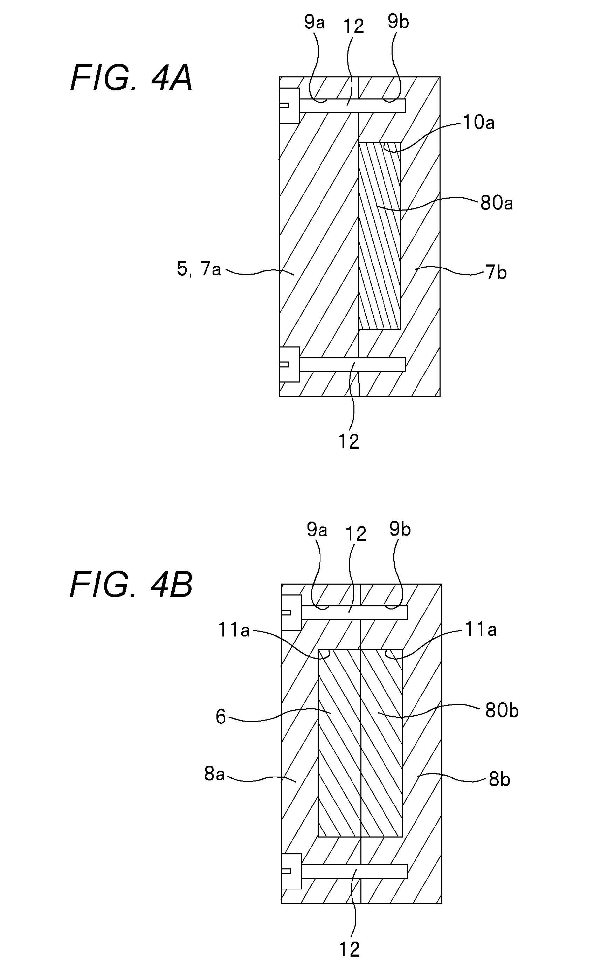

A connection plate 8b forming the other part of the second connector 8 is formed in a plate-like shape similar to the above-described slider 8a. A recess groove 11a for accommodating the femur frame 80b is provided on the surface of the connection plate 8b facing the slider 8a. Similarly, another recess groove 11a for accommodating the second arm member is provided in the surface of the slider 8a facing the connection plate 8b (see FIG. 4B). Further, screw holes 9b corresponding to the screw holes 9a of the slider 8a are provided in multiple positions of the connection plate 8b (four positions near four corners in this example). It is noted that, in the second connector 8, a connection plate may be provided to the second arm member 6, and a slider may be provided to the femur frame 80b.

As illustrated in FIG. 4A, the one connection plate 7a and the other connection plate 7b accommodating the waist frame 80a in the recess groove 10a are connected to each other by screwing screws 12 from the screw holes 9a of the one connection plate 7a to the screw holes 9b of the other connection plate 7b. Thereby, the first arm member 5 is fixed to the waist frame 80a.

On the other hand, as illustrated in FIG. 4B, the slider 8a accommodating the second arm member 6 in the recess groove 11a and the connection plate 8b accommodating the femur frame 80b in the recess groove 11a are connected to each other by screwing the screws 12 from the screw holes 9a of the slider 8a to the screw holes 9b of the connection plate 8b. Then, while the femur frame 80b and the connection plate 8b are fixed to each other, the slider 8a and the second arm member 6 are slidable with respect to each other. As described above, the slider 8a is connected by the screws 12 to the connection plate 8b fixed to the femur frame 80b. Therefore, the second arm member 6 is slidable with respect to the femur frame 80b. That is, the second arm member 6 is connected to the femur frame 80b in a slidable manner in the longitudinal direction.

The above-described connection structure allows the motor 4, the first arm member 5, and the second arm member 6 of the movement assist device 1 to be attached to and removed from the hip joint equipment 80 and 80'. In this way, in the example illustrated in FIG. 1A, the first arm member 5 is connected to the waist of the equipped person M via the waist frame 80a. Furthermore, the second arm member 6 is connected to the femur part of the right leg F1 of the equipped person M via the femur frame 80b. Further, in the example illustrated in FIG. 1B, the first arm member 5 is connected to the waist of the equipped person M via the waist frame 80a. Furthermore, the second arm member 6 is connected to the femur part of the right leg F1 of the equipped person M via the femur frame 80b'.

< Manual switch >

The manual switch 20 is attached to the waist of the equipped person M via the waist band B1 as illustrated in FIG. 1A and FIG. 1B.

The manual switch 20 is manually operated by a person who helps the equipped person M (for example, a medical worker such as a physical therapist) when the equipped person M makes a walking movement. This allows for the assist of the walking movement of the equipped person M by the movement assist device 1.

Specifically, the manual switch 20 has a first switch 20a, a second switch 20b, and a third switch 20c, as illustrated in FIG. 5. When manually operated, the first switch 20a and the second switch 20b are configured to output the trigger signal that triggers the driving of the motor 4. Among the above, the first switch 20a functions as a kick-out switch for assisting the kick-out movement by the right leg F1 of the equipped person M. The first switch 20a outputs a first trigger signal. This first trigger signal is a signal for driving the motor 4 so that the two arm members 5 and 6 expand (that is, driving the motor 4 so that the motor 4 rotates in the direction to cause the two arm members 5 and 6 to expand). The second switch 20b functions as a shake-out switch for assisting the shake-out movement by the right leg F1 of the equipped person M. The second switch 20b outputs a second trigger signal. This second trigger signal is a signal for driving the motor 4 so that the two arm members 5 and 6 bend (that is, driving the motor 4 so that the motor 4 rotates in the direction to cause the two arm members 5 and 6 to bend). The third switch 20c functions as a stop switch. For example, the equipped person M manually operates the third switch 20c when stopping its both legs (the right leg F1 and a left leg F2). Thereby, the third switch 20c outputs a trigger signal for causing the motor 4 to stop driving.

It is noted that the term "expand" of the arm members 5 and 6 refers to the revolution of the second arm member 6 (or the relative revolution of the first arm member 5 and the second arm member 6) to the direction so that the angle between the first arm member 5 and the second arm member 6 (the angle between the waist frame 80a and the femur frame 80b) increases. In contrast, the term "bend" refers to the revolution of the second arm member 6 (or the relative revolution of the first arm member 5 and the second arm member 6) to the direction so that the above-described angle decreases.

< Microphone >

A not-shown microphone is attached to the waist of the equipped person M via the waist band B1, for example. It is noted that the microphone may be fixed to other position than the waist of the equipped person M (for example, the chest and the like).

The voice of the person who helps the equipped person M is inputted to the microphone when the equipped person M makes a walking movement. The voice to be inputted may include, for example, the voice expressing the movement such as "kick-out", "shake-out", or the like, the call such as "one two, one two", and the hand clapping sound.

< Sensor unit >

The sensor unit 30 is set to an insole I1 of the footwear S1 of the right leg F1 side (see FIG. 1A) as illustrated in FIG. 6. It is noted that the sensor unit 30 may be set to other position of the footwear S1 than the insole I1 (for example, a shoe sole and the like). The sensor unit 30 has a first pressure-sensitive sensor 31a, a fixed resistor 32, a second pressure-sensitive sensor 31b, and a fixed resistor 33.

The first pressure-sensitive sensor 31a is set to the position in the insole I1 corresponding to the thenar eminence of the sole of the right leg F1. It is noted that the first pressure-sensitive sensor 31a may be set to the position in the insole I1 corresponding to other part than the thenar eminence of the sole of the right leg F1. The resistance of the first pressure-sensitive sensor 31a changes depending on the force (the pressure) applied to the position corresponding to the thenar eminence of the sole of the right leg F1. This allows the first pressure-sensitive sensor 31a to detect the force applied to that thenar eminence. In this example, as the first pressure-sensitive sensor 31a, used is a pressure-sensitive sensor in which the resistance increases according to the increase in the force applied to the position corresponding to the thenar eminence of the sole of the right leg F1. It is noted that, as the first pressure-sensitive sensor 31a, used may be a pressure-sensitive sensor in which the resistance decreases according to the increase in the force applied to the position corresponding to the thenar eminence of the sole of the right leg F1. The fixed resistor 32 has substantially a constant resistance and is connected in series to the first pressure-sensitive sensor 31a. That is, when substantially a constant voltage is applied to the first pressure-sensitive sensor 31a and the fixed resistor 32, the ratio between the resistance of the first pressure-sensitive sensor 31a and the resistance of the fixed resistor 32 changes depending on the force applied to the first pressure-sensitive sensor 31a. As a result, the voltage of the connection node of the first pressure-sensitive sensor 31a and the fixed resistor 32 changes. This voltage is inputted to a microcontroller 36 (described later) of a sensor module 35 as an output voltage Vout1 representing the detection result by the first pressure-sensitive sensor 31a. The sensor module 35 is set to the position in the footwear S1 corresponding to the instep of the right leg F1, for example.

The second pressure-sensitive sensor 31b is set to the position in the insole I1 corresponding to the heel of the sole of the right leg F1. It is noted that the second pressure-sensitive sensor 31b may be set to the position in the insole I1 corresponding to other part than the heel of the sole of the right leg F1. The resistance of the second pressure-sensitive sensor 31b changes depending on the force (the pressure) applied to the position corresponding to the heel of the sole of the right leg F1. This allows the second pressure-sensitive sensor 31b to detect the force applied to that heel. In this example, as the second pressure-sensitive sensor 31b, used is a pressure-sensitive sensor in which the resistance increases according to the increase in the force applied to the position corresponding to the heel of the sole of the right leg F1. It is noted that, as the second pressure-sensitive sensor 31a, used may be a pressure-sensitive sensor in which the resistance decreases according to the increase in the force applied to the position corresponding to the heel of the sole of the right leg F1. The fixed resistor 33 has substantially a constant resistance and is connected in series to the second pressure-sensitive sensor 31b. That is, when substantially a constant voltage is applied to the second pressure-sensitive sensor 31b and the fixed resistor 33, the ratio between the resistance of the second pressure-sensitive sensor 31b and the resistance of the fixed resistor 33 changes depending on the force applied to the second pressure-sensitive sensor 31b. As a result, the voltage of the connection node of the second pressure-sensitive sensor 31b and the fixed resistor 33 changes. This voltage is inputted to the microcontroller 36 (described later) of the sensor module 35 as an output voltage Vout2 representing the detection result by the second pressure-sensitive sensor 31b.

It is noted that, hereafter, the first pressure-sensitive sensor 31a and the second pressure-sensitive sensor 31b are collectively referred to as a pressure-sensitive sensor 31.

< Functional configuration of the sensor module and the control module >

Next, the conceptual functional configuration of the sensor module 35 and the control module 50 will be described below by referring to FIG. 7.

As illustrated in FIG. 7, the sensor module 35 has the microcontroller 36, an antenna 37 for radio communication with the above-described control module 50 (for transmitting information to the control module 50), and a battery for the driving power supply (not shown).

The microcontroller 36 is inputted with the output voltage Vout1 representing the detection result of the first pressure-sensitive sensor 31a and the output voltage Vout2 representing the detection result of the second pressure-sensitive sensor 31b.

Here, the applied voltage is denoted as Vin, the resistance of the first pressure-sensitive sensor 31a is denoted as R1, and the resistance of the fixed resistor 32 is denoted as R2, and the output voltage Vout1 is expressed by the following equation (1).

$$Vout1 = Vin \cdot R2 / (R1 + R2) \ ...(1)$$

As described above, as the first pressure-sensitive sensor 31a, used is the pressure-sensitive sensor in which the resistance increases according to the increase in the force applied to the position corresponding to the thenar eminence of the sole of the right leg F1. Therefore, in response to the increase of the force applied to the position corresponding to the thenar eminence, the resistance R1 of the first pressure-sensitive sensor 31a increases and thus the output voltage Vout1 decreases.

Also, the applied voltage is denoted as Vin, the resistance of the second pressure-sensitive sensor 31b is denoted as R3, and the resistance of the fixed resistor 33 is denoted as R4, and the output voltage Vout2 is expressed by the following equation (2).

$$Vout2 = Vin \cdot R4 / (R3 + R4) \ ...(2)$$

As described above, as the second pressure-sensitive sensor 31b, used is the pressure-sensitive sensor in which the resistance increases according to the increase in the force applied to the position corresponding to the heel of the sole of the right leg F1. Therefore, in response to the increase of the force applied to the position corresponding to the heel, the resistance R3 of the second pressure-sensitive sensor 31b increases and thus the output voltage Vout2 decreases.

Further, when the value of the output voltage Vout1 is less than or equal to a threshold and the value of the output voltage Vout2 is less than or equal to a threshold, the microcontroller 36 determines that the force applied to (the thenar eminence and the heel of) the right leg F1 is greater than or equal to a desired value (a predetermined value), that is, the shoe sole of the footwear S1 is in contact with the ground at a proper force. Therefore, when the value of the output voltage Vout1 is less than or equal to the threshold (that is, the first pressure-sensitive sensor 31a is ON) and the value of the output voltage Vout2 is less than or equal to the threshold (that is, the second pressure-sensitive sensor 31b is ON), the microcontroller 36 outputs the first trigger signal.

On the other hand, when the value of the output voltage Vout1 is greater than the threshold and the value of the output voltage Vout2 is greater than the threshold, the microcontroller 36 determines that the force applied to (the thenar eminence and the heel of) the right leg F1 is less than the desired value (the predetermined value), that is, the shoe sole of the footwear S1 is floating. Therefore, when the value of the output voltage Vout1 is greater than the threshold (that is, the first pressure-sensitive sensor 31a is OFF) and the value of the output voltage Vout2 is greater than the threshold (that is, the second pressure-sensitive sensor 31b is OFF), the microcontroller 36 outputs the second trigger signal.

It is noted that, in the present embodiment, the first trigger signal is outputted when both values of the output voltage Vout1 and the output voltage Vout2 are less than or equal to the thresholds, and the second trigger signal is outputted when both values are greater than the thresholds. Instead, the first trigger signal may be outputted when one of the values of the output voltage Vout1 and the output voltage Vout2 (for example, the output voltage Vout2 of the heel side) is less than or equal to the threshold, and the second trigger signal may be outputted when this value is greater than the threshold.

Further, the microcontroller 36 transmits the first trigger signal and the second trigger signal to the control module 50 by the radio communication via the antenna 37.

The control module 50 is connected to the sensor module 35 so as to be able to perform radio communication. Furthermore, the control module 50 is communicably connected to the controller 60 via the cable. It is noted that the control module 50 may be communicably connected to the sensor module 35 via the cable and connected to the controller 60 so as to be able to perform radio communication. The control module 50 controls the controller 60 based on the detection result of the sensor 31 received by the radio communication with the sensor module 35 (the details will be described later).

Further, the controller 60 is communicably connected to the motor 4 via a not-shown cable. It is noted that the controller 60 may be connected to the motor 4 so as to be able to perform radio communication. The controller 60 performs the driving control of the motor 4 based on the control signal inputted from the control module 50.

As illustrated in FIG. 7, the control module 50 has an antenna 51 for performing radio communication with the sensor module 35 (receiving information from the sensor module 35), a microcontroller 52, and a battery of the driving power supply (not shown). The microcontroller 52 has a signal acceptor 53, a voice input unit 54, and a mode selector 55.

The voice input unit 54 is configured to output the first trigger signal and the second trigger signal that correspond to the voice inputted to the above-described microphone. For example, the voice input unit 54 outputs the first trigger signal in response to the utterance "kick-out" and outputs the second trigger signal in response to the utterance "shake-out". Further, for example, the voice input unit 54 outputs the first trigger signal in response to "one" in the call "one two, one two" and outputs the second trigger signal in response to "two". Further, for example, the voice input unit 54 outputs the first trigger signal in response to the odd-numbered hand clapping sound and outputs the second trigger signal in response to the even-numbered hand clapping sound.

The signal acceptor 53 receives the trigger signal transmitted from the sensor module 35 by the radio communication via the antenna 51 to accept this trigger signal. Further, the signal acceptor 53 accepts the trigger signal outputted from the switch 20 by the manual operation by the helper. Further, the signal acceptor 53 accepts the trigger signal outputted from the audio input 54 in response to the voice inputted to the microphone. The signal acceptor 53 corresponds to an example of the means for accepting the trigger signal.

The mode selector 55 selects one of the trigger signals that is to be accepted by the signal acceptor 53 in accordance with the operation of the not-shown selection switch and the like. That is, the mode selector 55 is configured to select any one of a "switch mode" in which the trigger signal by the manual switch 20 is used, a "voice mode" in which the trigger signal by the voice is used, and a "sensor mode" in which the trigger signal by the sensor 31 is used. When the "switch mode" is selected by the mode selector 55, the signal acceptor 53 accepts the trigger signal from the manual switch 22, while does not accept the trigger signals from the voice input unit 54 and the sensor module 35. The similar is applied when other mode is selected.

Upon accepting the trigger signal from the sensor module 35, from the manual switch 20, or from the voice input unit 54, the signal acceptor 53 outputs the accepted trigger signal to the controller 60.

The controller 60 has a driving controller 61 and a battery (not shown) for the driving power supply. The driving controller 61 is inputted with the trigger signal from the signal acceptor 53 and performs the driving control of the motor 4 based on the inputted trigger signal. Specifically, when inputted with the first trigger signal, the driving controller 61 drives the motor 4 so that the first arm member 5 and the second arm member 6 expand (that is, drives the motor 4 so that the motor 4 rotates in the direction to cause the two arm members 5 and 6 to expand). Further, when inputted with the second trigger signal, the driving controller 61 drives the motor 4 so that the first arm member 5 and the second arm member 6 bend (that is, drives the motor 4 so that the motor 4 rotates in the direction to cause the two arm members 5 and 6 to bend). The driving controller 61 corresponds to an example of the means for driving the motor.

< Operation of the movement assist device >

When the "switch mode" is selected by the mode selector 55, the helper assists the walking movement of the equipped person M by operating the manual switch 20. In response to the operation of the first switch 20a, the first trigger signal is outputted from the manual switch 20 to the signal acceptor 53. In response, the driving controller 61 drives the motor 4 so that the two arm members 5 and 6 expand. As a result, the movement assist device 1 assists the kick-out movement of the right leg F1 of the equipped person M. On the other hand, in response to the operation of the second switch 20b, the second trigger signal is outputted from the manual switch 20 to the signal acceptor 53. In response, the driving controller 61 drives the motor 4 so that the two arm members 5 and 6 bend. As a result, the movement assist device 1 assists the shake-out movement of the right leg F1 of the equipped person M. Therefore, by that the helper operates the first switch 20a and the second switch 20b of the manual switch 20 alternately at a proper timing, the movement assist device 1 alternately assists the kick-out movement and the shake-out movement of the right leg F1 of the equipped person M to assist the walking movement of the equipped person M. It is noted that, when stopping the assist, the helper operates the third switch 20c. Thereby, the trigger signal for causing the motor 4 to stop driving is outputted to the signal acceptor 53. In response, the driving controller 61 stops the driving of the motor 4.

When the "voice mode" is selected by the mode selector 55, the helper assists the walking movement of the equipped person M by the voice. In response that the voice corresponding to the kick-out operation (the voice of "kick-out", the voice of "one", the odd-numbered hand clapping sound, and the like) is inputted to the microphone, the first trigger signal is outputted from the voice input unit 54 to the signal acceptor 53. In response, the driving controller 61 drives the motor 4 so that the two arm members 5 and 6 expand. As a result, the movement assist device 1 assists the kick-out movement of the right leg F1 of the equipped person M. On the other hand, in response that the voice corresponding to the shake-out operation (the voice of "shake-out", the voice of "two", the even-numbered hand clapping sound, and the like) is inputted to the microphone, the second trigger signal is outputted from the voice input unit 54 to the signal acceptor 53. In response, the driving controller 61 drives the motor 4 so that the two arm members 5 and 6 bend. As a result, the movement assist device 1 assists the shake-out movement of the right leg F1 of the equipped person M. Therefore, by that the helper inputs the voice corresponding to the kick-out or the shake-out to the microphone alternately at a proper timing, the movement assist device 1 alternately assists the kick-out movement and the shake-out movement of the right leg F1 of the equipped person M to assist the walking movement of the equipped person M.

When the "sensor mode" is selected by the mode selector 55, the assist without the helper is allowed. It is noted that the helper may assist the walking movement of the equipped person M by controlling the contact to the ground of the footwear S1 of the equipped person M. As described above, when the shoe sole of the footwear S1 contacts with the ground at a proper force (when the first pressure-sensitive sensor 31a is ON and the second pressure-sensitive sensor 31b is ON), the first trigger signal is outputted from the microcontroller 36 to the signal acceptor 53. In response, the driving controller 61 drives the motor 4 so that the two arm members 5 and 6 expand. As a result, the movement assist device 1 assists the kick-out movement of the right leg F1 of the equipped person M. On the other hand, when the shoe sole of the footwear S1 floats from the ground (when the first pressure-sensitive sensor 31a is OFF and the second pressure-sensitive sensor 31b is OFF), the second trigger signal is outputted from the microcontroller 36 to the signal acceptor 53. In response, the driving controller 61 drives the motor 4 so that the two arm members 5 and 6 bend. As a result, the movement assist device 1 assists the shake-out movement of the right leg F1 of the equipped person M. When the equipped person M walks, the movements of grounding of the right leg F1, kick-out of the right leg F1 (floating, shake-out, grounding, and kick-out of the left leg F2), floating of the right leg F1, shake-out of the right leg F1, and grounding of the right leg F1 are repeated. By the above-described movement control, the movement assist device 1 alternately assists the kick-out movement and the shake-out movement of the right leg F1 of the equipped person M to assist the walking movement of the equipped person M.

< Advantages of the embodiment >

As described above, the movement assist device 1 of the present embodiment has the motor 4, the two arm members 5 and 6, the signal acceptor 53, and the driving controller 61. In response to the trigger that the signal acceptor 53 accepts the first trigger signal, the driving controller 61 drives the motor 4 so that the two arm members 5 and 6 expand.

This allows for adjustment of the operating timing of the motor 4 in accordance with the physical function (or the physical state) of the equipped person M by outputting the first trigger signal to the signal acceptor 53 at a proper timing. Therefore, the assist according to the physical function of the equipped person can be achieved.

Further, the expanding movement of the two arm members 5 and 6 corresponds to the kick-out movement when the movement assist device 1 is applied to the hip joint as seen in the present embodiment. In the walking movement, the kick-out is an important movement accompanied by a weight movement. Therefore, the adjustable timing of the kick-out movement allows for a more effective assist to the equipped person.

Further, in the present embodiment, in particular, in response to the trigger that the signal acceptor 53 accepts the second trigger signal, the driving controller 61 drives the motor so that the two arm members 5 and 6 bend. This allows for adjustment of the operation timing of the motor 4 for both expanding movement and the bending movement of the two arm members 5 and 6. This can further enhance the adaptation to the physical function of the equipped person.

Further, in the present embodiment, in particular, the motor 4 and the two arm members 5 and 6 is attachable to and removable from the hip joint equipment 80 and 80' used by the equipped person M. This allows for the following advantages. Specifically, in general, the equipment mounted with the actuator such as the motor is configured in an integrated manner. In this case, it cannot uniquely support the equipped persons M having the different physiques. This causes insufficient fitting ability of the equipment. Furthermore, there is likelihood that the function cannot be sufficiently performed due to the nonconformity of the equipment. Moreover, since it becomes difficult to use the existing equipment, which requires a purchase of new whole equipment and thus the cost increases. Furthermore, when it is used, the removal and attachment of the entire equipment are needed. Moreover, it takes more time and cost for the repairing and maintenance. In contrast, in the present embodiment, the above-described configuration allows for the use of the existing equipment that the equipped person M usually uses. Therefore, the movement assist device 1 of the present embodiment has a high fitting property and the equipped person is thus able to use the equipment fitted to its physique to obtain a sufficient assist of the motor driving. Further, the existing equipment can be utilized, which allows for the high versatility and the reduction in the cost. Further, the attachment and removal of the motor 4 and the arm members 5 and 6 only are needed for use, that is, the removal and attachment of the equipment are unnecessary, which can improve the convenience at the equipped person. Moreover, the repairing and the maintenance can be simplified.

Further, in the present embodiment, in particular, the movement assist device 1 has the switches 20a and 20b configured to output the first trigger signal and the second trigger signal according to the manual operation. This allows the helper assisting the equipped person M, for example, the medical worker such as the physical therapist to use the switches 20a and 20b to adjust the operation timing of the motor 4 by the manual operation. Therefore, the helper is able to perform the assist that is more suitable to the physical function of the equipped person.

Further, in the present embodiment, in particular, the movement assist device 1 has the voice input unit 54 configured to output the first trigger signal and the second trigger signal according to the inputted voice. This allows the helper to adjust the operation timing of the motor by the utterance, the hand clapping sound, and the like. Therefore, the helper is able to perform the assist that is more suitable to the physical function of the equipped person M.

Further, in the present embodiment, in particular, the movement assist device 1 has the sensor 31 configured to detect the force applied to the sole of the equipped person M and outputs the first trigger signal and the second trigger signal according to the detection result. Further, the sensor 31 outputs the first trigger signal to cause the two arm members 5 and 6 to expand when the sensor 31 detects the grounding of the right leg F1, thereby the movement assist device 1 assists the kick-out movement. On the other hand, the sensor 31 outputs the second trigger signal to cause the two arm members 5 and 6 to bend when the sensor 31 detects the floating of the right leg F1, thereby the movement assist device 1 assists the shake-out movement. This allows for the adjustment of the operating timing of the motor 4 according to the movement state of the right leg F1 of the equipped person M. Therefore, the walking assist that is more suitable to the physical function of the equipped person M can be achieved.

Further, in the present embodiment, in particular, the first connector 7 (7 a, 7b) and the second connector 8 (8a, 8b) are used to connect the two arm members 5 and 6 to the corresponding parts of the hip joint equipment 80 of the equipped person M. The second connector 8 connects the arm member 6 to the femur frame 80b so that the arm member 6 and the femur frame 80b are slidable with respect to each other along the longitudinal direction. Thereby, even when the rotation axis AX1 of the motor 4 and the joint axis AX2 of the connection part 80d of the femur frame 80b (FIG. 3) are slightly shifted to each other, the displacement of the arm member 6 to the connection part (equipment) 80d during revolving operation due to this axial shift can be absorbed by the relative slide of the arm member 6 and the femur frame 80b and thus the assist by the motor 4 can be performed.

Further, in the present embodiment, in particular, the movement assist device 1 has the manual switch 20, the voice input unit 54, and the sensor 31. The mode selector 55 selects any one of the "switch mode" in which the trigger signal by the manual switch 20 is used, the "voice mode" in which the trigger signal by the voice is used, and the "sensor mode" in which the trigger signal by sensor 31 is used. This allows for the selection of the proper "mode" according to the physical function of the equipped person M, the usage environment of the movement assist device 1, and so on. This can enhance the adaptation to the equipped person M and improve the convenience.

It is noted that, in the above-described embodiment, the movement assist device 1 is applied to the walking assist of the equipped person M who wears the hip joint equipment 80 on its one of the legs (the right leg F1 in the above-described example). However, without limitation, the movement assist device 1 may be applied to the equipped person who wears the equipment on its both legs, for example. Further, either one of the first pressure-sensitive sensor 31a and the second pressure-sensitive sensor 31b may be set as the sensor.

< Modified examples >

It is noted that the embodiment is not limited to the above. Various modifications are possible within the scope not departing from its spirit and technical concept. Such modified examples will be described below.

(1) The case where the pressure-sensitive sensors are arranged to both legs

While the sensor 31 is set to the right leg F1 of the equipped person M in the above-described embodiment, both legs of the equipped person M may be provided with the sensor. In this case, the signal acceptor 53 may control the motor driving to stop in response to the trigger that the first trigger signals are accepted from both of the two sensors.

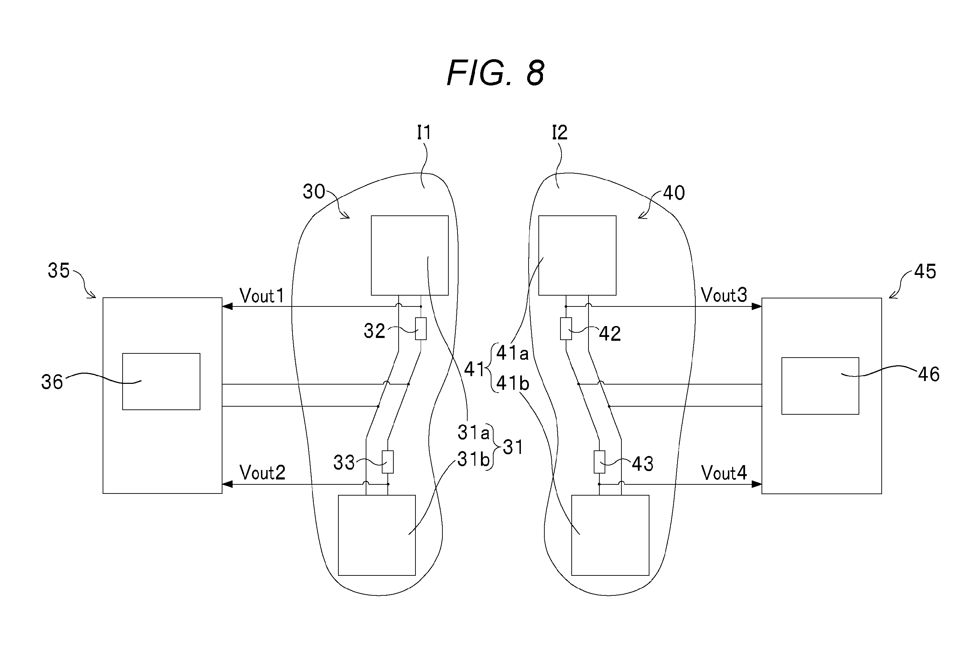

FIG. 8 illustrates an example of the configuration of the sensor units for both legs in the present modified example. As illustrated in FIG. 8, a sensor unit 40 is provided to the left leg F2 of the equipped person M similarly to the right leg F1 provided with the sensor unit 30. The sensor unit 40 is set to an insole I2 of the footwear of the left leg F2. The sensor unit 40 is configured similarly to the sensor unit 30, and has a first pressure-sensitive sensor 41a, a fixed resistor 42, a second pressure-sensitive sensor 41b, and a fixed resistor 43.

An output voltage Vout3 outputted by the first pressure-sensitive sensor 41a and an output voltage Vout4 outputted by the second pressure-sensitive sensor 41b are inputted to a microcontroller 46 of a sensor module 45.

When the values of the inputted output voltages Vout3 and Vout4 are less than or equal to a threshold, the microcontroller 46 determines that the shoe sole of the left leg F2 is in contact with the ground at a proper force, and outputs the first trigger signal. On the other hand, when the values of the output voltages Vout3 and Vout4 are greater than the threshold, the microcontroller 46 determines that the shoe sole of the left leg F2 is floating, and outputs the second trigger signal.

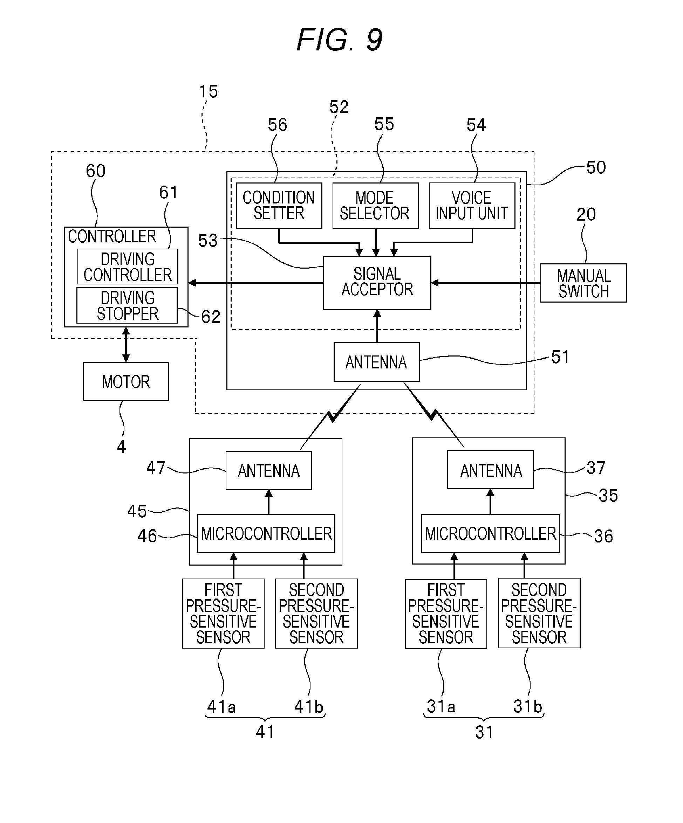

FIG. 9 illustrates the functional configuration of the movement assist device 1 in the present modified example. The functional block diagram illustrated in FIG. 9 is similar to the functional block diagram illustrated in FIG. 7 except that the pressure-sensitive sensor 41 and the sensor module 45 are added, that a condition setter 56 is added to the control module 50, and that a driving stopper 62 is added to the controller 60.

The driving stopper 62 is configured to stop the driving of the motor 4 in response to the trigger that the signal acceptor 53 accepts the first trigger signals from both of the pressure-sensitive sensor 31 and the pressure sensitive sensor 41. That is, under the selection of the "sensor mode" by the mode selector 55, upon accepting the first trigger signals from both of the first sensor 31 and the second sensor 41, the signal acceptor 53 outputs the first trigger signal and the second trigger signal to the controller 60. As a result, the driving stopper 62 stops the driving of the motor 4.

Thereby, it can be suppressed that the movement assist device 1 erroneously continues the walking assist when the equipped person M stops walking and has both legs F1 and F2 contact to the ground. Therefore, the movement assist device 1 is able to implement the proper walking assist according to the intention of the equipped person M.

Further, the condition setter 56 is configured to set the condition under which the driving stopper 62 stops the driving of the motor 4. That is, even when the equipped person M stops walking and has both legs F1 and F2 contact to the ground, there is likelihood that the distribution of the weight in the sole is different between the left and right legs due to the custom and the habit of the equipped person M. For example, in the case where the equipped person M stops walking in a position close to tiptoe standing, there may be a case where the weight is unlikely to be applied to the heels. In contrast, in the case where the equipped person M stops walking with substantially applying its weight on the heels only and substantially floating its tiptoes, there may be a case where the weight is unlikely to be applied to the tiptoes. In order to address such cases, the condition setter 56 sets the condition under which the driving stopper 62 stops the driving of the motor 4.

For example, when the equipped person M is the person who stops walking in the position close to tiptoe standing as described above, the condition setter 56 may set the condition so that the driving of the motor 4 stops in response to the trigger that both ON signals from the first pressure-sensitive sensors 31a and 41a are accepted. On the other hand, when the equipped person M is the person who stops walking with substantially applying its weight on the heels only and substantially floating its tiptoes as described above, the condition setter 56 may set the condition so that the driving of the motor 4 stops in response to the trigger that both ON signals from the second pressure-sensitive sensors 31b and 41b are accepted. This allows for the walking assist by changing the condition even in the above-described cases. Therefore, the adaptation to the equipped person M can be further enhanced.

(2) The case where the movement assist device is applied to knee joint equipment

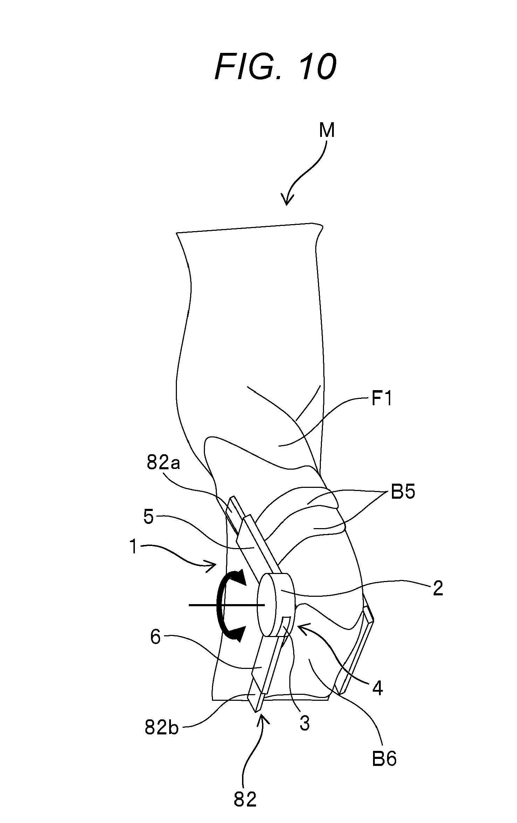

The present modified example is an example when the movement assist device 1 is applied to knee joint equipment. FIG. 10 illustrates an example of the present modified example.

As illustrated in FIG. 10, knee joint equipment 82 has a femur frame 82a extending along the thigh from the knee and a cruris frame 82b extending along the cruris from the knee in the right side of the right leg F1 of the equipped person M. The femur frame 82a is attached along the right side of the femur via two femur bands B5 mounted on the femur of the equipped person M. The upper end of the cruris frame 82b is connected to the lower end of the femur frame 82a, and the cruris frame 82b is able to revolve with respect to the femur frame 82a. The cruris frame 82b is attached along the longitudinal direction of the cruris via a support band B6 mounted under the knee of the cruris.

It is noted that, in the present modified example, the term "expand" refers to the revolution of the cruris frame 82b (the second arm member 6) (or the relative revolution of the femur frame 82a (the first arm member 5) and the cruris frame 82b (the second arm member 6)) to the direction so that the angle between the femur frame 82a and the cruris frame 82b (the angle of the backside of the body) increases. In contrast, the term "bend" refers to the revolution of the cruris frame 82b (the second arm member 6) (or the relative revolution of the femur frame 82a (the first arm member 5) and the cruris frame 82b (the second arm member 6)) to the direction so that the above-described angle decreases.

The first arm member 5 of the movement assist device 1 is connected to the femur of the right leg F1 of the equipped person M via the femur frame 82a of the knee joint equipment 82. The second arm member 6 is connected to the cruris of the right leg F1 via the cruris frame 82b. In the present modified example, the motor 4, the first arm member 5, and the second arm member 6 of the movement assist device 1 are configured to be attachable to and removable from the existing knee joint equipment 82 by the connection structure similar to the above-described embodiment.

Other configuration of the present modified example is basically similar to the above-described embodiment. That is, in response to the trigger that the signal acceptor 53 accepts the first trigger signal, the driving controller 61 drives the motor 4 so that the two arm members 5 and 6 expand. In response to the trigger that the signal acceptor 53 accepts the second trigger signal, the driving controller 61 drives the motor 4 so that the two arm members 5 and 6 bend. It is noted that the condition under which the microcontroller 36 outputs the first trigger signal and the second trigger signal under the "sensor mode" can be properly changed according to the configuration of the present modified example.

Therefore, the assist according to the physical function of the equipped person can be achieved and thus the advantages similar to the above-described embodiments can be obtained.

(3) The case where the movement assist device is applied to ankle joint equipment

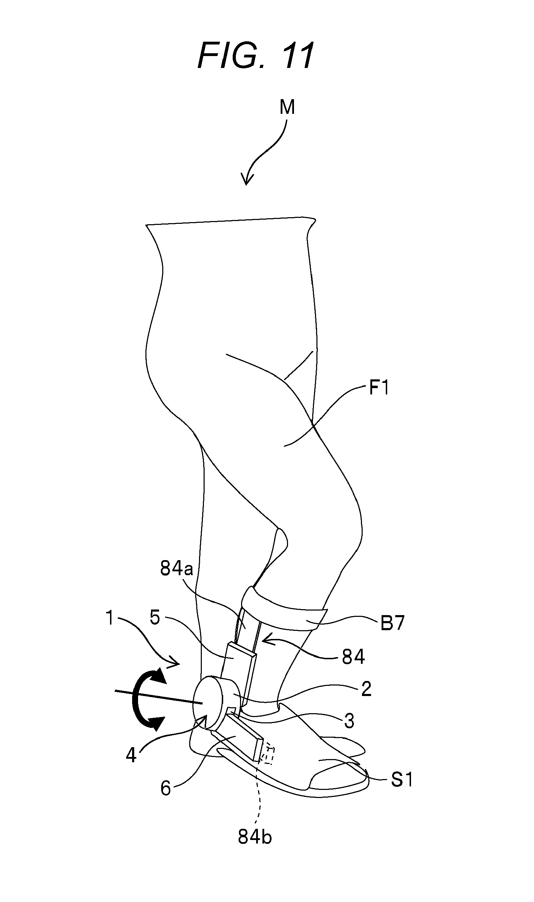

The present modified example is an example when the movement assist device 1 is applied to ankle joint equipment. FIG. 11 illustrates an example of the present modified example.

As illustrated in FIG. 11, ankle joint equipment 84 has a cruris frame 84a extending along the cruris from the ankle and an instep frame 84b extending along the side of the instep from the ankle in the right side of the right leg F1 of the equipped person M. The cruris frame 84a is attached along the right side from the ankle of the cruris via a cruris band B7 mounted on the cruris. One end of the instep frame 84b is connected to the lower end of the cruris frame 84a, and the instep frame 84b is able to revolve with respect to the cruris frame 84a. While the instep frame 84b is arranged inside the footwear S1 in this example, it may be arranged outside of the footwear S 1.

It is noted that, in the present modified example, the term "expand" refers to the revolution of the instep frame 84b (the second arm member 6) (or the relative revolution of the cruris frame 84a (the first arm member 5) and the instep frame 84b (the second arm member 6)) to the direction so that the angle between the cruris frame 84a and the instep frame 84b (the angle of the foreside of the body) increases. In contrast, the term "bend" refers to the revolution of the instep frame 84b (the second arm member 6) (or the relative revolution of the cruris frame 84a (the first arm member 5) and the instep frame 84b (the second arm member 6)) to the direction so that the above-described angle decreases.

The first arm member 5 of the movement assist device 1 is connected to the cruris of the right leg F1 of the equipped person M via the instep frame 84a of the ankle joint equipment 84. The second arm member 6 is connected to the instep of the right leg F1 via the instep frame 84b. In the present modified example, the motor 4, the first arm member 5, and the second arm member 6 of the movement assist device 1 are configured to be attachable to and removable from the existing ankle joint equipment 84 by the connection structure similar to the above-described embodiment.

Other configuration of the present modified example is basically similar to the above-described embodiment. That is, in response to the trigger that the signal acceptor 53 accepts the first trigger signal, the driving controller 61 drives the motor 4 so that the two arm members 5 and 6 expand. In response to the trigger that the signal acceptor 53 accepts the second trigger signal, the driving controller 61 drives the motor 4 so that the two arm members 5 and 6 bend. It is noted that the condition under which the microcontroller 36 outputs the first trigger signal and the second trigger signal under the "sensor mode" can be properly changed according to the configuration of the present modified example.

Therefore, the assist according to the physical function of the equipped person can be achieved and thus the advantages similar to the above-described embodiments can be obtained.

(4) The case where the movement assist device is applied to shoulder joint equipment

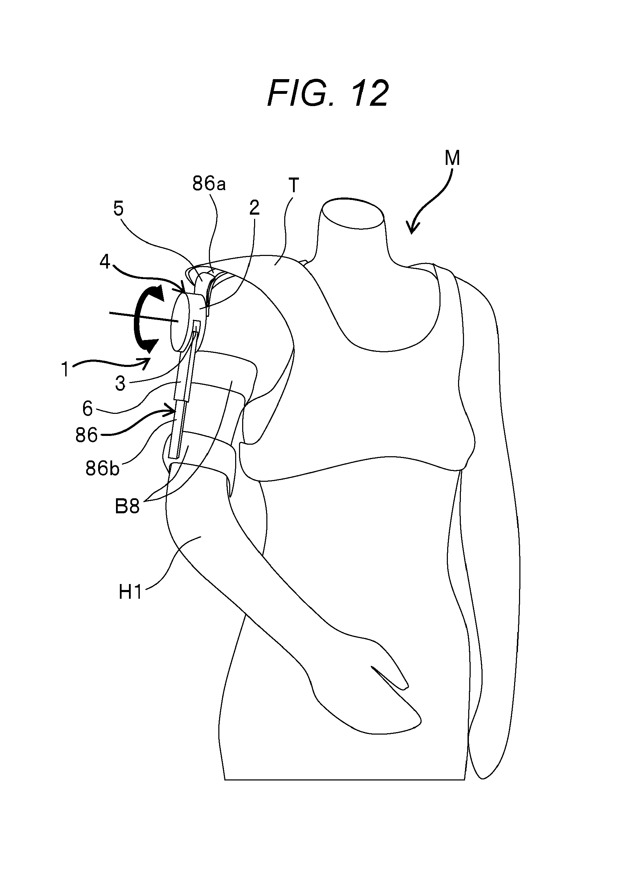

The present modified example is an example when the movement assist device 1 is applied to shoulder joint equipment. FIG. 12 illustrates an example of the present modified example.

As illustrated in FIG. 12, shoulder joint equipment 86 has an upper shoulder frame 86a curving and extending along the upper shoulder from the corner of the right shoulder of the equipped person M and an upper arm frame 86b extending along the upper arm from the corner. The upper shoulder frame 86a is fixed to the support T-shirts T put on the bust of the equipped person M via a not-shown attachment member. The upper end of the upper arm frame 86b is connected to the lower end of the upper shoulder frame 86a, and the upper arm frame 86b is able to revolve with respect to the upper shoulder frame 86a. The upper arm frame 86b is attached along the upper arm from the corner of the shoulder via upper arm bands B8 mounted on two parts, namely, the upper and lower parts of the upper arm.

It is noted that, in the present modified example, the term "expand" refers to the revolution of the upper arm frame 86b (the second arm member 6) (or the relative revolution of the upper shoulder frame 86a (the first arm member 5) and the upper arm frame 86b (the second arm member 6)) to the direction so that the angle between the upper shoulder frame 86a and the upper arm frame 86b (the angle of the foreside of the body) increases. In contrast, the term "bend" refers to the revolution of the upper arm frame 86b (the second arm member 6) (or the relative revolution of the upper shoulder frame 86a (the first arm member 5) and the upper arm frame 86b (the second arm member 6)) to the direction so that the above-described angle decreases.

The first arm member 5 of the movement assist device 1 is formed in a shape having a curve along the upper shoulder frame 86a. The first arm member 5 of the movement assist device 1 is connected to the right shoulder of the equipped person M via the upper shoulder frame 86a of the shoulder joint equipment 86. The second arm member 6 is connected to the upper arm via the upper arm frame 86b. Also in the present modified example, the motor 4, the first arm member 5, and the second arm member 6 of the movement assist device 1 are configured to be attachable to and removable from the existing shoulder joint equipment 86 by the connection structure similar to the above-described embodiment.

Other configuration of the present modified example is basically similar to the above-described embodiment. That is, in response to the trigger that the signal acceptor 53 accepts the first trigger signal, the driving controller 61 drives the motor 4 so that the two arm members 5 and 6 expand. In response to the trigger that the signal acceptor 53 accepts the second trigger signal, the driving controller 61 drives the motor 4 so that the two arm members 5 and 6 bend. It is noted that, in the present modified example, the "switch mode" or the "voice mode" is selected, but the "sensor mode" is not selected.

Therefore, the assist according to the physical function of the equipped person can be achieved and thus the advantages similar to the above-described embodiments can be obtained.

(5) The case where the movement assist device is applied to elbow joint equipment

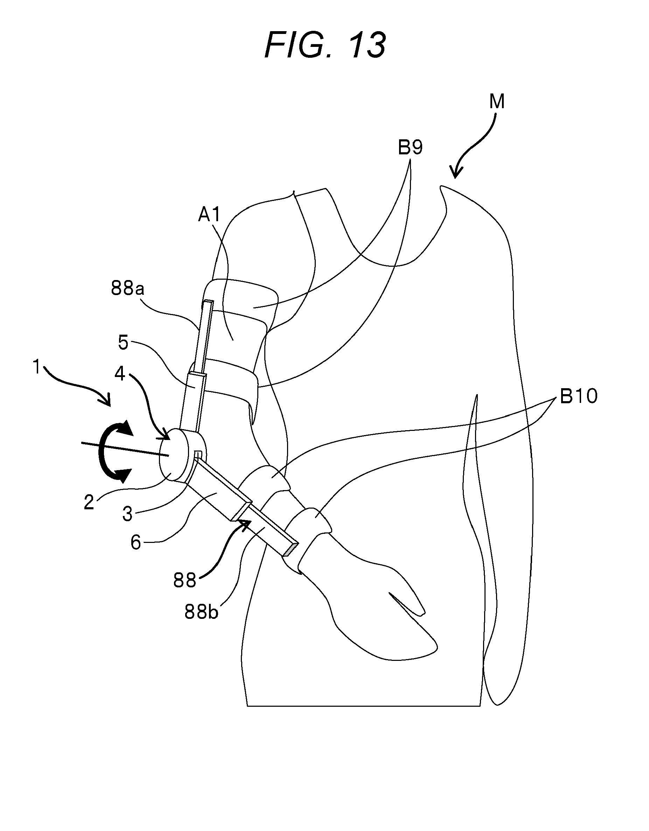

The present modified example is an example when the movement assist device is applied to elbow joint equipment. FIG. 13 illustrates an example of the present modified example.

As illustrated in FIG. 13, elbow joint equipment 88 has an upper arm frame 88a extending along the upper arm from the elbow of the right arm A1 of the equipped person M and a forearm frame 88b extending along the forearm from the elbow. The upper arm frame 88a is attached along the upper arm via upper arm bands B9 mounted on two parts, namely, the upper and lower parts of the upper arm of the right arm A1. The upper end of the forearm frame 88b is connected to the lower end of the upper arm frame 88a, and the forearm frame 88b is able to revolve with respect to the upper arm frame 88a. The forearm frame 88b is attached along the forearm via forearm bands B10 mounted on two parts, namely, the upper and lower parts of the forearm.

It is noted that, in the present modified example, the term "expand" refers to the revolution of the forearm frame 88b (the second arm member 6) (or the relative revolution of the upper arm frame 88a (the first arm member 5) and the forearm frame 88b (the second arm member 6)) to the direction so that the angle between the upper arm frame 88a and the forearm frame 88b (the angle of the foreside of the body) increases. In contrast, the term "bend" refers to the revolution of the forearm frame 88b (the second arm member 6) (or the relative revolution of the upper arm frame 88a (the first arm member 5) and the forearm frame 88b (the second arm member 6)) to the direction so that the above-described angle decreases.

The first arm member 5 of the movement assist device 1 is connected to the upper arm of the right arm A1 of the equipped person M via the upper arm frame 88a of the elbow joint equipment 88. The second arm member 6 is connected to the forearm of the right arm A1 via the forearm frame 88b. Also in the present modified example, the motor 4, the first arm member 5, and the second arm member 6 of the movement assist device 1 are configured to be attachable to and removable from the existing elbow joint equipment 88 by the connection structure similar to the above-described embodiment.

Other configuration of the present modified example is basically similar to the above-described embodiment. That is, in response to the trigger that the signal acceptor 53 accepts the first trigger signal, the driving controller 61 drives the motor 4 so that the two arm members 5 and 6 expand. In response to the trigger that the signal acceptor 53 accepts the second trigger signal, the driving controller 61 drives the motor 4 so that the two arm members 5 and 6 bend. It is noted that, in the present modified example, the "switch mode" or the "voice mode" is selected, but the "sensor mode" is not selected.

Therefore, the assist according to the physical function of the equipped person can be achieved and thus the advantages similar to the above-described embodiments can be obtained.

(6) The case where the movement assist device is applied to wrist joint equipment

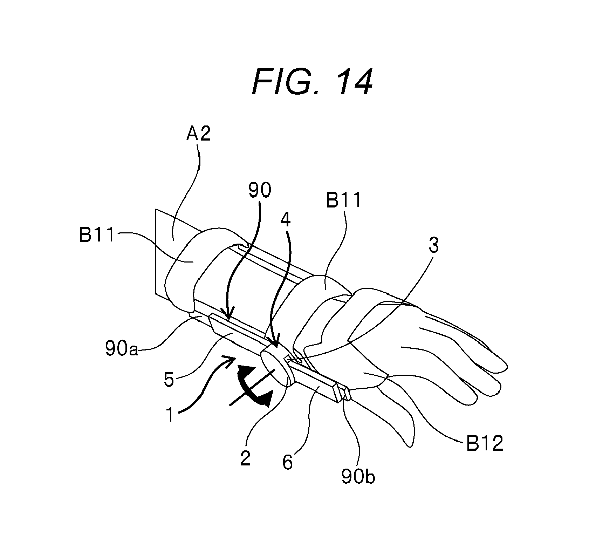

The present modified example is an example when the movement assist device is applied to wrist joint equipment. FIG. 14 illustrates an example of the present modified example.

As illustrated in FIG. 14, wrist joint equipment 90 has a forearm frame 90a extending along the forearm from the wrist in the inside of the left arm A2 of the equipped person M and a wrist frame 90b extending from the wrist to the side of the thumb. The forearm frame 90a is attached along the forearm via forearm bands B11 mounted on two parts, namely, the upper and lower parts of the forearm. One end of the wrist frame 90b is connected to one end of the forearm frame 90a, and the wrist frame 90b is able to revolve with respect to the forearm frame 90a. The wrist frame 90b is attached along the side from the wrist to the thumb via a back-of-the-hand supporter B12 mounted on the back of the hand from the wrist.

It is noted that, in the present modified example, the term "expand" refers to the revolution of the wrist frame 90b (the second arm member 6) (or the relative revolution of the forearm frame 90a (the first arm member 5) and the wrist frame 90b (the second arm member 6)) to the direction so that the angle between the forearm frame 90a and the wrist frame 90b (the angle of the inside of the body) increases. In contrast, the term "bend" refers to the revolution of the wrist frame 90b (the second arm member 6) (or the relative revolution of the forearm frame 90a (the first arm member 5) and the wrist frame 90b (the second arm member 6)) to the direction so that the above-described angle decreases.

The first arm member 5 of the movement assist device 1 is connected to the forearm of the left arm A2 of the equipped person M via the forearm frame 90a of the wrist joint equipment 90. The second arm member 6 is connected to the hand of the left arm A2 via the wrist frame 90b. Also in the present modified example, the motor 4, the first arm member 5, and the second arm member 6 of the movement assist device 1 are configured to be attachable to and removable from the existing wrist joint equipment 90 by the connection structure similar to the above-described embodiment.

Other configuration of the present modified example is basically similar to the above-described embodiment. That is, in response to the trigger that the signal acceptor 53 accepts the first trigger signal, the driving controller 61 drives the motor 4 so that the two arm members 5 and 6 expand. In response to the trigger that the signal acceptor 53 accepts the second trigger signal, the driving controller 61 drives the motor 4 so that the two arm members 5 and 6 bend. It is noted that, in the present modified example, the "switch mode" or the "voice mode" is selected, but the "sensor mode" is not selected.

Therefore, the assist according to the physical function of the equipped person can be achieved and thus the advantages similar to the above-described embodiments can be obtained.

Further, other than the above-described examples, the approaches of the above-described embodiment and respective modified examples may be utilized in a proper combination thereof.

In addition, although not exemplified in detail here, the above-described embodiment and respective modified examples may be implemented with various modifications added within the scope not departing from their spirit.

It is noted that the signal acceptor 53 corresponds to an example of the signal accepting unit. The driving controller 61 corresponds to an example of the driving control unit. The voice input unit 54 corresponds to an example of the voice input unit. The mode selector 55 corresponds to an example of the mode selection unit.

The driving stopper 62 corresponds to an example of the driving stop unit. The condition setter 56 corresponds to an example of the condition setting unit.

Further, the movement assist device of the present embodiment may be the following first to tenth movement assist devices. The first movement assist device is a movement assist device for assisting physical movement of an equipped person and has a motor including a stator and a rotator, two arm members connected to each of the stator and the rotator and configured to be connected to a predetermined part of a body of the equipped person, a signal acceptor configured to accept a trigger signal that triggers a driving of the motor, and a driving controller configured to drive the motor in a direction so that the two arm members expand in response to a trigger that the signal acceptor accepts a first one of the trigger signal.

In the second movement assist device in the first movement assist device, the driving controller is configured to drive the motor in a direction so that the two arm members bend in response to a trigger that the signal acceptor accepts second one of the trigger signal.

In the third movement assist device in the first or second movement assist device, the motor and the two arm members are configured to be attachable to and removable from equipment used by the equipped person.

In the third movement assist device, the fourth movement assist device has switches configured to output the first trigger signal and the second trigger signal according to a manual operation.

In the third or fourth movement assist device, the fifth movement assist device has a voice input unit configured to output the first trigger signal and the second trigger signal according to an inputted voice.

In any one of the third to fifth movement assist devices, the sixth movement assist device has a sensor configured to detect a force applied to a sole of the equipped person and output the first trigger signal and the second trigger signal according to the detection result.

In the seventh movement assist device in the sixth movement assist device, the sensors are arranged to both legs of the equipped person, the movement assist device further has a driving stopper configured to stop the driving of the motor in response to a trigger that the signal acceptor accepts the first trigger signals from both of the two sensors.

In the seventh movement assist device, the eighth movement assist device has a condition setter configured to set a condition under which the driving stopper stops the driving of the motor.

In any one of the third to eighth movement assist devices, the ninth movement assist device has a first connector configured to fix one of the two arm members to a corresponding part of the equipment and a second connector configured to connect the other of the two arm members to a corresponding part of the equipment so that they are slidable with respect to any one of the arm members and the equipment in a longitudinal direction.

In any one of the second to ninth movement assist devices, the tenth movement assist device has switches configured to output the first trigger signal and the second trigger signal according to a manual operation, a voice input unit configured to output the first trigger signal and the second trigger signal according to the inputted voice, a sensor configured to detect a force applied to a sole of the equipped person and output the first trigger signal and the second trigger signal according to the detection result, and a mode selector configured to select any one of a switch mode in which the trigger signal by the switches is used, a voice mode in which the trigger signal by the voice is used, and a sensor mode in which the trigger signal by the sensor is used.

The foregoing detailed description has been presented for the purposes of illustration and description. Many modifications and variations are possible in light of the above teaching. It is not intended to be exhaustive or to limit the subject matter described herein to the precise form disclosed. Although the subject matter has been described in language specific to structural features and/or methodological acts, it is to be understood that the subject matter defined in the appended claims is not necessarily limited to the specific features or acts described above. Rather, the specific features and acts described above are disclosed as example forms of implementing the claims appended hereto.

**Claims**

1. A movement assist device (1) comprising:

    a motor (4) including a stator (2) and a rotator (3); two arm members (5, 6) connected to each of the stator and the rotator and configured to be connected to a predetermined part of a body of an equipped person (M); a signal accepting unit (53) configured to accept a trigger signal that triggers a driving of the motor; and a driving control unit (61) configured to drive the motor so that the two arm members expand in response to a trigger that the signal accepting unit accepts a first one of the trigger signal.

2. The movement assist device according to claim 1, wherein the driving control unit is configured to drive the motor so that the two arm members bend in response to a trigger that the signal accepting unit accepts a second one of the trigger signal.

3. The movement assist device according to claim 1, wherein the motor and the two arm members are configured to be attachable to and removable from equipment used by the equipped person.

4. The movement assist device according to claim 2, wherein the motor and the two arm members are configured to be attachable to and removable from equipment used by the equipped person.

5. The movement assist device according to claim 4 further comprising a switch (20a, 20b) configured to output the first trigger signal and the second trigger signal according to a manual operation.

6. The movement assist device according to claim 4 or 5 further comprising a voice input unit (54) configured to output the first trigger signal and the second trigger signal according to an inputted voice.

7. The movement assist device according to any one of claims 4 to 6 further comprising a sensor (31a, 31b) configured to detect a force applied to a sole of the equipped person and output the first trigger signal and the second trigger signal according to the detection result.

8. The movement assist device according to any one of claims 3 to 7 further comprising:

    a first connector (7) configured to fix one of the two arm members to a corresponding part of the equipment; and a second connector (8) configured to connect the other of the two arm members to a corresponding part of the equipment so that the other of the arm members and the equipment are slidable with respect to each other in a longitudinal direction.

9. The movement assist device according to any one of claims 2 to 4 and 8 further comprising:

    a switch (20a, 20b) configured to output the first trigger signal and the second trigger signal according to a manual operation; a voice input unit (54) configured to output the first trigger signal and the second trigger signal according to an inputted voice; a sensor (31a, 31b) configured to detect a force applied to a sole of the equipped person and output the first trigger signal and the second trigger signal according to the detection result; and a mode selection unit (55) configured to select any one of a switch mode in which the trigger signal by the switches is used, a voice mode in which the trigger signal by the voice input unit is used, and a sensor mode in which the trigger signal by the sensor is used.

10. The movement assist device according to claim 7 or 9, wherein the sensor includes two sensors arranged respectively to both legs, of the equipped person,

the movement assist device further including a driving stop unit (62) configured to stop the driving of the motor in response to a trigger that the signal accepting unit accepts the first trigger signals from both of the two sensors.

11. The movement assist device according to claim 10 further comprising a condition setting unit (56) configured to set a condition under which the driving stop unit stops the driving of the motor.

12. The movement assist device according to claim 5 or 9,
   wherein the switches include a stop switch configured to output a trigger signal for causing the motor to stop driving according to a manual operation, and wherein the driving control unit is configured to stop the motor in response to a trigger that the signal accepting unit accepts a trigger signal for causing the motor to stop driving.

13. The movement assist device according to claim 7 or 9,
   wherein the sensor includes
   a first pressure-sensitive sensor (31a) configured to detect a force applied to a thenar eminence of the sole of the equipped person, and
   a second pressure-sensitive sensor (31b) configured to detect a force applied to a heel of the sole of the equipped person, and
   wherein, while the first trigger signal is outputted when both of the force applied to the thenar eminence of the sole of the equipped person and the force applied to the heel of the sole of the equipped person are greater than or equal to a predetermined value, the second trigger signal is outputted when both of the forces are smaller than the predetermined value.

FIG. 1B

FIG. 1A

# FIG. 2

# FIG. 3

FIG. 4A

FIG. 4B

*FIG. 5*

*FIG. 6*

EP 2 856 997 A2

# FIG. 7

# FIG. 8

# FIG. 9

# FIG. 10

# FIG. 11

# FIG. 12

# FIG. 13

# FIG. 14

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP S61228854 B